(19) Europäisches Patentamt
European Patent Office
Office européen des brevets

(11) Veröffentlichungsnummer: **0 102 924**
**B1**

(12) **EUROPÄISCHE PATENTSCHRIFT**

(45) Veröffentlichungstag der Patentschrift: **06.04.88**

(21) Anmeldenummer: **83810385.1**

(22) Anmeldetag: **26.08.83**

(51) Int. Cl.⁴: **A 01 N 47/36,** C 07 D 239/46, C 07 D 239/52, C 07 D 251/16 // C07C143/78

# (54) N-Azidophenylsulfonyl-N'-pyrimidinyl- und -triazinylharnstoffe.

(30) Priorität: **01.09.82 CH 5196/82**

(43) Veröffentlichungstag der Anmeldung: **14.03.84 Patentblatt 84/11**

(45) Bekanntmachung des Hinweises auf die Patenterteilung: **06.04.88 Patentblatt 88/14**

(84) Benannte Vertragsstaaten: **AT BE CH DE FR GB IT LI NL SE**

(56) Entgegenhaltungen:
**EP-A-0 001 515**

**TETRAHEDRON LETTERS, No. 45, 1977, OXFORD (GB), Seiten 3973-76, THOMAS McC. PATERSON et al.: "Deazidation of aryl azides with hydrazine hydrate. A new synthesis of 3-aminoindazole"**

(73) Patentinhaber: **CIBA- GEIGY AG, Klybeckstrasse 141, CH- 4002 Basel (CH)**

(72) Erfinder: **Thummel, Rudolf Carl, Route d'Alle 424, CH- 2892 Courgenay (CH)**
Erfinder: **Föry, Werner, Dr., Benkenstrasse 65, CH- 4054 Basel (CH)**

**EP 0 102 924 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue, herbizid wirksame und pflanzenwuchsregulierende N-Azidophenylsulfonyl-N'-pyrimidinyl- und -triazinylharnstoffe, Verfahren zu ihrer Herstellung, sie als Wirkstoffe enthaltende Mittel und deren Verwendung zum Bekämpfen von Unkräutern, vor allem selektiv in Nutzpflanzenkulturen oder zum Regulieren und Hemmen des Pflanzenwachstums.

Die erfindungsgemässen N-Azidophenylsulfonyl-N'-pyrimidinyl- und -triazinylharnstoffe entsprechen der allgemeinen Formel I

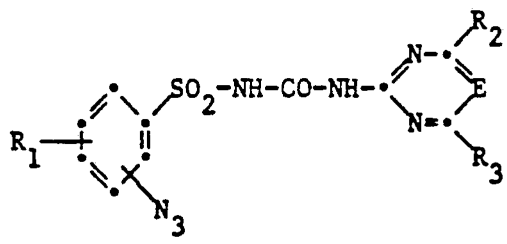

(I),

worin

E Stickstoff oder die Methinbrücke,

$R_1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,

$R_2$ $C_1$-$C_3$-Alkyl, $C_1$-$C_3$-Alkoxy oder $C_1$-$C_3$-Halogenalkoxy,

$R_3$ Wasserstoff, Halogen, $C_1$-$C_3$-Alkyl, $C_1$-$C_3$-Halogenalkyl, $C_1$-$C_3$-Alkoxy, $C_1$-$C_3$-Halogenalkoxy, $C_2$-$C_5$-Alkoxyalkoxy oder $-NR_4R_5$ bedeuten, wobei

$R_4$ für Wasserstoff oder Methyl und

$R_5$ für Wasserstoff, Methyl, Äthyl oder Methoxy stehen, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in der europäischen Patentpublikation Nr. 1515, beschrieben.

In den Definitionen ist unter Alkyl geradkettiges oder verzweigtes Alkyl zu verstehen: z. B. Methyl, Äthyl, n-Propyl, i-Propyl oder die vier isomeren Butyl.

Unter Alkoxy ist zu verstehen: Methoxy, Äthoxy, n-Propyloxy, i-Propyloxy und die vier isomeren Butyloxyreste, insbesondere aber Methoxy, Äthoxy oder i-Propoxy.

Beispiele für Alkylthio sind Methylthio, Äthylthio, n-Propylthio, i-Propylthio und n-Butylthio, insbesondere aber Methylthio und Äthylthio.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl, n-Propylsulfonyl und n-Butylsulfonyl, insbesondere aber Methylsulfonyl und Äthylsulfonyl.

Unter Halogen in den Definitionen sowie in Halogenalkyl und -alkoxy sind Fluor, Chlor und Brom, vorzugsweise jedoch Fluor und Chlor zu verstehen.

Entsprechend sind unter Halogenalkyl bzw. Halogenalkylteilen von oben definierten Substituenten beispielsweise zu verstehen: Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel I sind die Verbindungen bevorzugt, in denen entweder

a) der Azidorest in 2-Stellung zur Sulfonylgruppe steht, oder

b) $R_1$ Wasserstoff ist, oder
c) $R_2$ und $R_3$ zusammen nicht mehr als 3 Kohlenstoffatome enthalten, oder
d) $R_2$ und $R_3$ ausgewählt sind aus der Gruppe Methyl, Methoxy, Dimethylamino, Difluormethoxy und 2,2,2-Trifluoräthoxy, oder
e) $R_1$ $C_1$-$C_4$-Alkoxycarbonyl bedeutet.

Eine weiter bevorzugte Untergruppe von Verbindungen der Formel I bilden diejenigen Verbindungen, in denen der Rest $R_1$ in 2-Stellung zur Sulfonylgruppe steht und $C_1$-$C_4$-Alkoxycarbonyl bedeutet und $R_2$ und $R_3$ ausgewählt sind aus der Gruppe Methyl, Methoxy, Difluormethoxy, Dimethylamino und 2,2,2-Trifluoräthoxy und zusammen nicht mehr als 3 Kohlenstoffatome enthalten.

Aus dieser Gruppe sind ganz besonders bevorzugt die Verbindungen der Formel I, in denen $R_2$ und $R_3$ für Methyl, Methoxy oder Difluormethoxy stehen.

Als bevorzugte Einzelverbindungen sind zu nennen:
N-(2-Azidophenylsulfonyl)-N'-(4-methoxy-6-methyl-1,3,5-triazin-2-yl)-harnstoff,
N-(2-Methoxycarbonyl-5-azidophenylsulfonyl)-N'-(4-difluormethoxy-6-methyl-pyrimidin-2-yl)-harnstoff und
N-(2-Azidophenylsulfonyl)-N'-(4-methoxy-6-methyl-pyrimidin-2-yl)-harnstoff.

Die Herstellung der Verbindungen der Formel I erfolgt im allgemeinen in einem inerten organischen Lösungsmittel.

Nach einem ersten Verfahren werden die Verbindungen der Formel I erhalten, indem man ein Azidophenylsulfonamid der Formel II

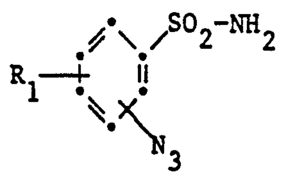

(II),

worin $R_1$ die unter Formel I gegebene Bedeutung hat, in Gegenwart einer Base mit einem N-Pyrimidinyl- oder -triazinylcarbamat der Formel III

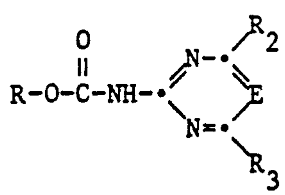

(III),

worin E, $R_2$ und $R_3$ die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein Azidophenylsulfonylisocyanat der Formel IV

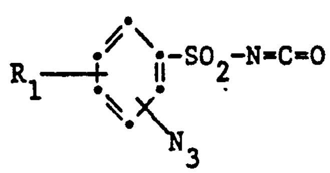

(IV),

worin $R_1$ die unter Formel I gegebene Bedeutung hat, gegebenenfalls in Gegenwart einer Base, mit einem Amin der Formel V

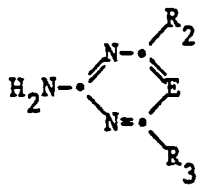

(V),

worin $R_2$ und $R_3$ die unter Formel I gegebene Bedeutung haben, umsetzt.

Weiterhin kann man die Verbindungen der Formel I auch erhalten, indem man ein N-Azidophenylsulfonylcarbamat der Formel VI

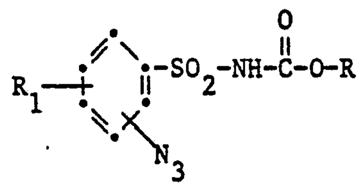

(VI),

worin $R_1$ die unter Formel I gegebene Bedeutung hat und R für Phenyl, Alkyl oder substituiertes Phenyl steht, mit einem Amin der oben angegebenen Formel V umsetzt.

Schliesslich werden die Verbindungen der Formel I auch hergestellt, indem man einen Aminosulfonylharnstoff der Formel VII

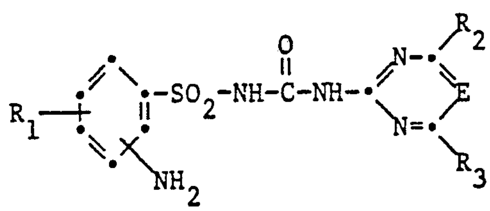

(VII),

worin E, $R_1$, $R_2$ und $R_3$ die unter Formel I gegebene Bedeutung haben, diazotiert und die Azidogruppe einführt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Ausgangsprodukte der Formeln III, V und VII sind bekannt oder können nach bekannten Methoden hergestellt werden.

Neue Fluoralkoxy-aminopyrimidine und -triazine der Formel V und ihre Herstellung sowie die Herstellung entsprechender Verbindungen der Formel II daraus werden in der europäischen Patentanmeldung 70 804 beschrieben.

Die neuen Verbindungen der Formeln IV und VI werden nach bekannten Methoden aus den Azidophenylsulfonamiden der Formel II hergestellt. Diese Verbindungen wurden speziell für die Synthese der Wirkstoffe der Formel I entwickelt und bilden daher einen Bestandteil der Erfindung.

Die Azidophenylsulfonamide der Formel II sind teilweise bekannt, vergleiche Monatshefte der Chemie, 81, 970-980 (1950).

Die neuen Azidophenylsulfonamide der Formel II wurden speziell für die Synthese der Verbindungen der Formel I sowie die Zwischenprodukte der Formeln IV und VI entwickelt.

Die Verbindungen der Formel II werden im allgemeinen erhalten, indem man analoge Nitrophenylsulfonamide nach bekannten Methoden, wie katalytische Reduktion mit Wasserstoff, Reduktion mit komplexen Metallhydriden, Reduktion mit Kombinationen von Metallen und Säuren, Reduktion mit Amalgamen und wässrigen Alkoholen oder Reduktion mit Alkalisulfiden, zu den Aminoverbindungen reduziert und diese in bekannter Weise diazotiert und in die Azidoverbindungen der Formel IIa überführt. Ähnliche Verfahren sind beispielsweise in Monatshefte der Chemie, 81, 970-980 (1950), oder in Houben-Weyl, Methoden der organischen Chemie, 4. Aufl., Bd.10/3, 781-836 beschrieben.

In analoger Weise können die Zwischenprodukte der Formel VII durch Reduktion aus entsprechenden Nitrophenylsulfonyl-harnstoffen gewonnen werden. Solche Nitro-phenyl-sulfonyl-harnstoffe sind entweder bekannt oder sie werden in Anlehnung an bekannte Methoden erhalten.

Die Umsetzungen zu Verbindungen der Formel I werden vorteilhafterweise in aprotischen, inerten, organischen Lösungsmitteln vorgenommen, wie Methylenchlorid, Tetrahydrofuran, Acetonitril, Dioxan, Toluol.

Die Reaktionstemperaturen liegen vorzugsweise zwischen -20°C und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln, in denen sie sich nicht gut lösen, wie Äther, aromatischen Kohlenwasserstoffen oder chlorierten Kohlenwasserstoffen, gereinigt werden.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

So wird beispielsweise bei Sojapflanzen nach Behandlung mit Verbindungen der Formel I eine vermehrte Schotenbildung am Haupttrieb der Pflanzen beobachtet, wobei gleichzeitig eine Gewichtszunahme der Schoten feststellbar ist.

Bei monokotylen Pflanzen, z. B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr der Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise

hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ , wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder Ka-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

H. Stache, "Tensid-Taschenbuch" 2. Aufl.,
C. Hanser Verlag, München, Wien, 1981.
M. + J. Ash, "Encyclopedia of Surfactants," Vol. I-III,
Chemical Publishing Co., New York, 1980-1981.

Die pestiziden Zubereitungen enthalten in der Regel 0,1 bis 95 %, insbesondere 0,1 bis 80 %, Wirkstoff der Formel I, 1 bis 99 % eines festen oder flüssigen Zusatzstoffes und 0 bis 25 %, insbesondere 0,1 bis 25 % eines

Tensides.

Insbesondere setzen sich bevorzugte Formulierungen folgendermassen zusammen: (% = Gewichtsprozent)

| | |
|---|---|
| Emulgierbare Konzentrate | |
| Aktiver Wirkstoff: | 1 bis 20 %, bevorzugt 5 bis 10 % |
| oberflächenaktives Mittel: | 5 bis 30 %, bevorzugt 10 bis 20 % |
| flüssiges Trägermittel: | 50 bis 94 %, vorzugsweise 70 bis 85 %. |
| Stäube | |
| Aktiver Wirkstoff: | 0,1 bis 10 %, vorzugsweise 0,1 bis 1 % |
| festes Trägermittel: | 99,9 bis 90 %, vorzugsweise 99,9 bis 99 %. |
| Suspensions-Konzentrate | |
| Aktiver Wirkstoff: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |
| Benetzbare Pulver | |
| Aktiver Wirkstoff: | 0,5 bis 90 %, vorzugsweise 1 bis 80 % |
| oberflächenaktives Mittel | 0,5 bis 20 %, vorzugsweise 1 bis 15 % |
| festes Trägermittel: | 5 bis 95 %, vorzugsweise 15 bis 90 %. |
| Granulate | |
| Aktiver Wirkstoff | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

**Beispiel 1**: N-(2-Azidophenylsulfonyl)-N'-(4-methoxy-6-methyl-1,3,5-triazin-2-yl)-harnstoff.

Zu einer Mischung von 3,4 g N-(2-Aminophenylsulfonyl)-N'-(4-methoxy-6-methyl-1,3,5-triazin-2-yl)-harnstoff, 10 g Eis und 4 ml 36 %-iger Salzsäure lässt man bei einer Temperatur von 0 bis 5° langsam eine Lösung von 1,0 g Natriumnitrit in 4 ml Wasser zutropfen. Nachdem das Reaktionsgemisch für weitere 2 Stunden bei 0° gerührt worden ist, setzt man tropfenweise eine Lösung von 1 g Natriumazid in 4 ml Wasser zu, und lässt die Temperatur auf 20° ansteigen. Die Reaktionsmischung wird dreimal mit je 20 ml Methylenchlorid extrahiert. Die vereinigten organischen Phasen werden getrocknet und eingedampft. Aus dem Rückstand erhält man durch Umkristallisieren 1,0 g N-(2-Azidophenylsulfonyl)-N'-(4-methoxy-6-methyl-1,3,5-triazin-2-yl)-harnstoff, Zers.175°.

**Beispiel 2**: N-(2-Azidophenylsulfonyl)-N'-(4,6-dimethoxy-pyrimidin-2-yl)-harnstoff.

Zu einer Mischung von 3,5 g N-(2-Aminophenylsulfonyl)-N'-dimethoxy-pyrimidin-2-yl)-harnstoff, 10 g Eis und 4 ml 36 %-iger Salzsäure lässt man bei einer Temperatur von 0° bis 5° langsam eine Lösung von 1,0 g Natriumnitrit in 4 ml Wasser zutropfen. Nachdem das Reaktionsgemisch für weitere 2 Stunden bei 0° gerührt worden ist, setzt man tropfenweise eine Lösung von 1 g Natriumazid in 4 ml Wasser zu und lässt die Temperatur auf 20° ansteigen. Die Reaktionsmischung wird dreimal mit je 20 ml Methylenchlorid extrahiert. Die vereinigten organischen Phasen werden getrocknet und eingedampft. Aus dem Rückstand erhält man durch Umkristallisieren 0,52 g N-(2-Azidophenylsulfonyl)-N'-(4,6-dimethoxy-pyrimidin-2-yl)-harnstoff, Zers. 190°.

**Beispiel 3**: N-(2-Azidophenylsulfonyl)-N'-(4-methoxy-6-methyl-pyrimidin-2-yl)-harnstoff.

Zu einer Mischung von 3,4 g N-(2-Aminophenylsulfonyl)-N'-(4-methoxy-6-methyl-pyrimidin-2-yl)-harnstoff, 10 g Eis und 4 ml 36 %-iger Salzsäure lässt man bei einer Temperatur von 0 bis 5° langsam eine Lösung von 1,0 g

Natriumnitrit in 4 ml Wasser zutropfen. Nachdem das Reaktionsgemisch für weitere 2 Stunden bei 0° gerührt worden ist, setzt man tropfenweise eine Lösung von 1 g Natriumazid in 4 ml Wasser zu und lässt die Temperatur auf 20° ansteigen. Die Reaktionsmischung wird dreimal mit je 20 ml Methylenchlorid extrahiert. Die vereinigten organischen Phasen werden getrocknet und eingedampft. Aus dem Rückstand erhält man durch Umkristallisieren 0,85 g (N-(2-Azidophenylsulfonyl)-N'-(4-methoxy-6-methyl-pyrimidin-2-yl)-harnstoff, Zers.178-179°.

**Beispiel 4**: N-(4-Azidophenylsulfonyl)-N'-(4-methoxy-6-methyl-pyrimidin-2-yl)-harnstoff.

Eine Lösung von 2,0 g 4-Azidophenylsulfonamid und 1,6 ml 1,5-Diaza-bicyclo(5,4,0)undec-5-en in 20 ml Dioxan wird bei 20-25°C tropfenweise mit einer Lösung von 2,9 g N-(4-Methoxy-6-methyl-pyrimidin-2-yl)-phenylcarbamat in 10 ml Dioxan versetzt und für 3 Stunden bei der gleichen Temperatur gerührt. Anschliessend wird das Reaktionsgemisch mit 200 ml Wasser aufgenommen und mit Salzsäure bis auf einen pH-Wert von 2 angesäuert. Das ausgefällte Produkt wird abgetrennt, mit Äthylacetat gewaschen und getrocknet. Man erhält so in 67 %-iger Ausbeute N-(4-Azidophenylsulfonyl)-N'-(4-methoxy-6-methyl-pyrimidin-2-yl)-harnstoff als grünliches Kristallisat, Smp. 192°C.

In analoger Weise erhält man die in den anschliessenden Tabellen aufgelisteten Zwischen- und Endprodukte.

**Tabelle 1**:

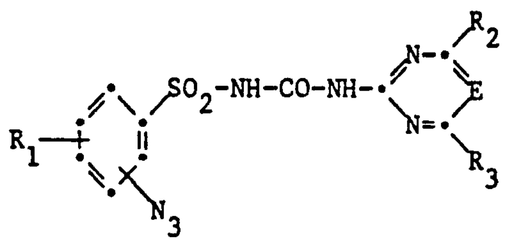

| Nr. | $R_1$ | Stellung der Azidogruppe | $R_2$ | $R_3$ | E | phys.Daten |
|---|---|---|---|---|---|---|
| 1.1 | H | 2 | $CH_3$ | $OCH_3$ | N | Smp.:174°C (Zers.) |
| 1.2 | H | 2 | $OCH_3$ | $OCH_3$ | N | |
| 1.3 | H | 2 | $OCH_3$ | $-N(CH_3)_2$ | N | |
| 1.4 | H | 2 | $OCH_3$ | $-OCH_2-CF_3$ | N | |
| 1.5 | H | 2 | $OCH_3$ | $OCH_3$ | CH | Smp.:190°C (Zers.) |
| 1.6 | H | 2 | $CH_3$ | $OCH_3$ | CH | Smp.:178-179°(Zers.) |
| 1.7 | H | 2 | $CH_3$ | $CH_3$ | CH | |
| 1.8 | H | 2 | $CH_3$ | $OCHF_2$ | CH | |
| 1.9 | 4-$OCH_3$ | 3 | $CH_3$ | $OCH_3$ | N | |
| 1.10 | 4-$OCH_3$ | 3 | $CH_3$ | $OCH_3$ | CH | Smp.:177°C (Zers.) |
| 1.11 | H | 3 | $CH_3$ | $OCH_3$ | N | |
| 1.12 | H | 3 | $CH_3$ | $OCH_3$ | CH | Smp.:190°C (Zers.) |
| 1.13 | H | 4 | $CH_3$ | $OCH_3$ | N | |
| 1.14 | H | 4 | $CH_3$ | $OCH_3$ | CH | Smp.:192°C (Zers.) |
| 1.15 | 2-$CH_3$ | 5 | $OCH_3$ | $CH_3$ | N | Smp.:170-173° |
| 1.16 | 6-Cl | 2 | $OCH_3$ | $CH_3$ | CH | Smp.:165° (Zers.) |
| 1.17 | 2-$COOCH_3$ | 5 | $CH_3$ | $OCH_3$ | N | |
| 1.18 | 2-$COOCH_3$ | 5 | $CH_3$ | $CH_3$ | N | |
| 1.19 | 2-$COOCH_3$ | 5 | $OCH_3$ | $OCH_3$ | N | |
| 1.20 | 2-$COOCH_3$ | 5 | $OCH_3$ | $N(CH_3)_2$ | N | |
| 1.21 | 2-$COOCH_3$ | 5 | $OCH_3$ | $OCH_2-CH_3$ | N | |
| 1.22 | 2-$COOCH_3$ | 5 | $OCH_3$ | $OCH_2-CF_3$ | N | |
| 1.23 | 2-$COOCH_3$ | 5 | $C_2H_5$ | $OCH_3$ | N | |
| 1.24 | 2-$COOCH_3$ | 5 | $OC_2H_5$ | $OC_2H_5$ | N | |
| 1.25 | 2-$COOCH_3$ | 5 | $CH_3$ | $CH_3$ | CH | |
| 1.26 | 2-$COOCH_3$ | 5 | $CH_3$ | $OCH_3$ | CH | |
| 1.27 | 2-$COOCH_3$ | 5 | $CH_3$ | $OCHF_2$ | CH | |

**Tabelle 1**:

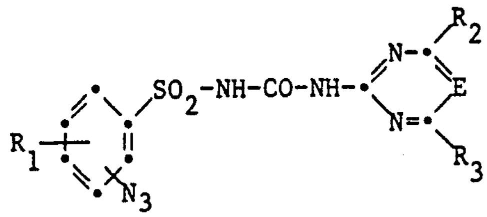

| Nr. | $R_1$ | Stellung der Azidogruppe | $R_2$ | $R_3$ | E | phys. Daten |
|---|---|---|---|---|---|---|
| 1.28 | 2-$COOCH_3$ | 5 | $OCH_3$ | $OCH_3$ | CH | |
| 1.29 | 2-$COOCH_3$ | 5 | $OCH_3$ | $OCHF_2$ | CH | |
| 1.30 | 2-$COOCH_3$ | 5 | $OCHF_2$ | $OCHF_2$ | CH | |
| 1.31 | 2-$COOCH_3$ | 5 | $OCH_3$ | Cl | CH | |
| 1.32 | 2-$COOCH_3$ | 5 | $OCH_3$ | F | CH | |
| 1.33 | 2-$COOCH_3$ | 5 | $OCH_2F$ | $OCH_2$ | CH | |
| 1.34 | 2-$COOCH_3$ | 5 | $OCH_3$ | $OCF_2$-$CHF_2$ | CH | |
| 1.35 | 2-$COOCH_3$ | 5 | $CH_3$ | $OCF_2$-$CHF_2$ | CH | |
| 1.36 | 2-$CH_3$ | 5 | $CH_3$ | $OCH_3$ | N | |
| 1.37 | 2-$CH_3$ | 5 | $CH_3$ | $CH_3$ | N | |
| 1.38 | 2-$CH_3$ | 5 | $OCH_3$ | $OCH_3$ | N | |
| 1.39 | 2-$CH_3$ | 5 | $OCH_3$ | $N(CH_3)_2$ | N | |
| 1.40 | 2-$CH_3$ | 5 | $OCH_3$ | $OCH_2$-$CH_3$ | N | |
| 1.41 | 2-$CH_3$ | 5 | $OCH_3$ | $OCH_2$-$CF_3$ | N | |
| 1.42 | 2-$CH_3$ | 5 | $C_2H_5$ | $OCH_3$ | N | |
| 1.43 | 2-$CH_3$ | 5 | $OC_2H_5$ | $OC_2H_5$ | N | |
| 1.44 | 2-$CH_3$ | 5 | $CH_3$ | $CH_3$ | CH | |
| 1.45 | 2-$CH_3$ | 5 | $CH_3$ | $OCH_3$ | CH | |
| 1.46 | 2-$CH_3$ | 5 | $CH_3$ | $OCHF_2$ | CH | |
| 1.47 | 2-$CH_3$ | 5 | $OCH_3$ | $OCH_3$ | CH | |
| 1.48 | 2-$CH_3$ | 5 | $OCH_3$ | $OCHF_2$ | CH | |
| 1.49 | 2-$CH_3$ | 5 | $OCHF_2$ | $OCHF_2$ | CH | |
| 1.50 | 2-$CH_3$ | 5 | $OCH_3$ | Cl | CH | |
| 1.51 | 2-$CH_3$ | 5 | $OCH_3$ | F | CH | |
| 1.52 | 2-$CH_3$ | 5 | $OCH_2F$ | $OCH_3$ | CH | |
| 1.53 | 2-$CH_3$ | 5 | $OCH_3$ | $OCF_2$-$CHF_2$ | CH | |
| 1.54 | 2-$CH_3$ | 5 | $CH_3$ | $OCF_2$-$CHF_2$ | CH | |

**Tabelle 2**:

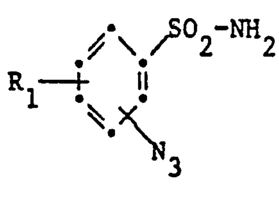

| Nr. | $R_1$ | Stellung der Azidogruppe | phys. Daten |
|---|---|---|---|
| 2.1 | H | 3 | Smp.:128-129°C |
| 2.2 | H | 4 | Smp.:118-119°C |
| 2.3 | 4-$OCH_3$ | 3 | Smp.128-130°C |
| 2.4 | H | 2 | Zers. bei 189°C |
| 2.5 | 2-$CH_3$ | 5 | |
| 2.6 | 2-$COOCH_3$ | 5 | |

**Formulierungsbeispiele**

**Beispiel 5**: Formulierungsbeispiele für Wirkstoffe der Formel I (% = Gewichtsprozent)

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel indem der Wirkstoff mit dem Träger vermischt und auf einer geeigneten Mühle vermahlen wird.

| d) Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |
| Äthylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %-ige wässrige Formaldehyd-Lösung | 0,2 % | 0,2 % |
| Silikonöl in Form einer 75 %-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5 % |
| Isopropylamin | 1 % |
| Octylphenolpolyäthylenglykoläther (78 Mol AeO) | 3 % |
| Wasser | 91 % |

**Beispiel 6**: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturium officinalis, Agrostis tenuis, Stellaria media und Digitaria sanguinalis. Die Versuchsgefässe werden anschliessend in einer Klimakammer bei 20°C, einer Beleuchtung von ca. 20 000 Lux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem folgenden Maßstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:
Konzentration der Wirkstoffemulsion: 70,8 ppm

| Testpflanze | Nasturtium | Stellaria | Agrostis | Digitaria |
|---|---|---|---|---|
| Wirkstoff Nr. | | | | |
| 1.1 | 2 | - | - | - |
| 1.5 | 1 | 2 | 1 | 1 |
| 1.6 | 2 | 2 | 2 | 2 |

- : nicht geprüft

**Beispiel 7**: Herbizidwirkung nach dem Auflaufen der Pflanzen (Kontaktwirkung)

Eine Anzahl Unkräuter und Kulturpflanzen, sowohl monokotyle wie dikotyle, wurden nach dem Auflaufen, im 4- bis 6-Blattstadium mit einer wässrigen Wirkstoffdispersion von 4 kg AS/ha gespritzt und dann bei 24° bis 26° und 45-60 % relativer Luftfeuchtigkeit gehalten. 15 Tage nach der Behandlung wird der Versuch ausgewertet. In diesem Versuch zeigen sie mit den Wirkstoffen der Formel I behandelten Pflanzen starke, irreversible Schädigungen.

**Beispiel 8**: Wuchshemmung bei tropischen Bodendecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt werden.

**Beispiel 9**: Wuchsregulierung an Sojabohnen

In Kunststoffbehältern mit einem Erde-Torf-Sandgemisch im Verhältnis 6 : 3 : 1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten am Haupttrieb.

**Beispiel 10**: Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zur unbehandelten Kontrolle eine Verringerung des Neuzuwachses (60-90 % der Kontrolle), sowie teilweise eine Zunahme der Stengeldurchmesser auf.

**Beispiel 11**: Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

**Patentansprüche** für die Vertragsstaaten BE, CH, DE, FR, GB, IT, LI, NL, SE.

1. N-Azidophenylsulfonyl-N'-pyrimidinyl- und -triazinylharnstoffe der Formel I

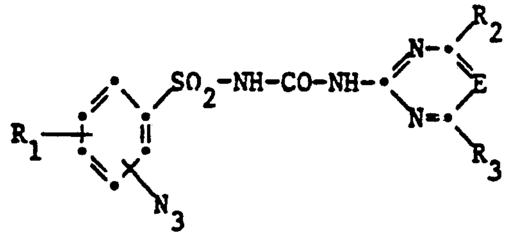

(I),

worin

E Stickstoff oder die Methinbrücke,

$R_1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,

$R_2$ $C_1$-$C_3$-Alkyl, $C_1$-$C_3$-Alkoxy oder $C_1$-$C_3$-Halogenalkoxy,

$R_3$ Wasserstoff, Halogen, $C_1$-$C_3$-Alkyl, $C_1$-$C_3$-Halogenalkyl, $C_1$-$C_3$-Alkoxy, $C_1$-$C_3$-Halogenalkoxy, $C_2$-$C_5$-Alkoxyalkoxy oder $-NR_4R_5$ bedeuten, wobei

$R_4$ für Wasserstoff oder Methyl und

$R_5$ für Wasserstoff, Methyl, Äthyl oder Methoxy stehen, sowie die Salze dieser Verbindungen.

2. Verbindungen gemäss Anspruch 1, dadurch gekennzeichnet, dass der Azidorest in 2-Stellung zur Sulfonylgruppe steht.

3. Verbindungen gemäss Anspruch 1, dadurch gekennzeichnet, dass $R_1$ Wasserstoff ist.

4. Verbindungen gemäss Anspruch 1, dadurch gekennzeichnet, dass $R_2$ und $R_3$ zusammen nicht mehr als 3 Kohlenstoffatome enthalten.

5. Verbindungen gemäss Anspruch 1, dadurch gekennzeichnet, dass $R_2$ und $R_3$ ausgewählt sind aus der Gruppe Methyl, Methoxy, Dimethylamino, Difluormethoxy und 2,2,2-Trifluoräthoxy.

6. Verbindungen gemäss Anspruch 1, dadurch gekennzeichnet, dass $R_1$ $C_1$-$C_4$-Alkoxycarbonyl bedeutet.

7. Verbindungen gemäss Anspruch 1, dadurch gekennzeichnet, dass der Rest $R_1$ in 2-Stellung zur Sulfonylgruppe steht und $C_1$-$C_4$-Alkoxycarbonyl bedeutet und $R_2$ und $R_3$ ausgewählt sind aus der Gruppe Methyl, Methoxy, Difluormethoxy, Dimethylamino und 2,2,2-Trifluoräthoxy und zusammen nicht mehr als 3 Kohlenstoffatome enthalten.

8. Verbindungen gemäss Anspruch 7, dadurch gekennzeichnet, dass $R_2$ und $R_3$ für Methyl, Methoxy oder Difluormethoxy stehen

9. N-(2-Methoxycarbonyl-5-azidophenylsulfonyl)-N'-(4-difluormethoxy-6-methyl-pyrimidin-2-yl)-harnstoff gemäss Anspruch 1.

10. N-(2-Azidophenylsulfonyl)-N'-(4-methoxy-6-methyl-1,3,5-triazin-2-yl)-harnstoff gemäss Anspruch 1.

11. N-(2-Azidophenylsulfonyl)-N'-(4-methoxy-6-methyl-pyrimidin-2-yl)-harnstoff gemäss Anspruch 1.

12. Verfahren zur Herstellung der Verbindungen der Formel I, Anspruch 1, dadurch gekennzeichnet, dass man ein Azidophenylsulfonamid der Formel II

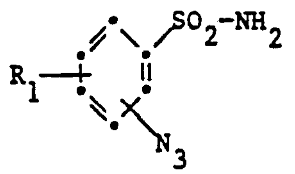

(II),

worin $R_1$ die unter Formel I gegebene Bedeutung hat, in Gegenwart einer Base mit einem N-Pyrimidinyl- oder -triazinylcarbamat der Formel III

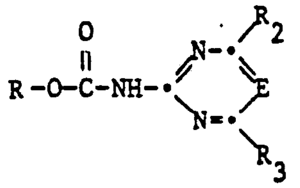

(III),

worin E, $R_2$ und $R_3$ die unter Formel I gegebene Bedeutung haben und R für Phenyl, Alkyl oder substituiertes Phenyl steht, umsetzt und gegebenenfalls in ihre Salze überführt.

13. Verfahren zur Herstellung der Verbindungen der Formel I, Anspruch 1, dadurch gekennzeichnet, dass man ein Azidophenylsulfonylisocyanat der Formel IV

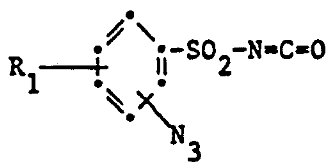

(IV),

worin $R_1$ die unter Formel I gegebene Bedeutung hat, gegebenenfalls in Gegenwart einer Base, mit einem Amin der Formel V

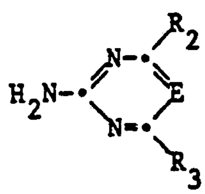

(V),

worin $R_2$ und $R_3$ die unter Formel I gegebene Bedeutung haben, umsetzt und gegebenenfalls in ihre Salze überführt.

14. Verfahren zur Herstellung der Verbindungen der Formel I, Anspruch 1, dadurch gekennzeichnet, dass man ein N-Azidophenylsulfonylcarbamat der Formel VI

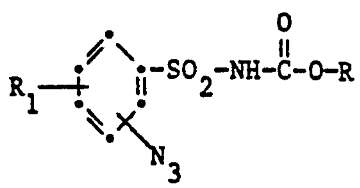

(VI),

worin $R_1$ die unter Formel I gegebene Bedeutung hat und R für Phenyl, Alkyl oder substituiertes Phenyl steht, mit einem Amin der oben angegebenen Formel V umsetzt und gegebenenfalls in ihre Salze überführt.

15. Verfahren zur Herstellung der Verbindungen der Formel I, Anspruch 1, dadurch gekennzeichnet, dass man einen Aminosulfonylharnstoff der Formel VII

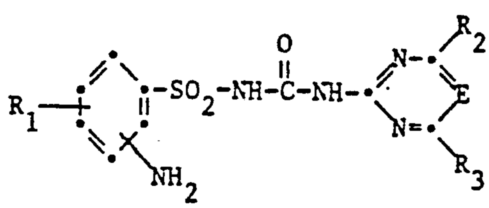

(VII),

worin E, $R_1$, $R_2$ und $R_3$ die unter Formel I gegebene Bedeutungen haben, diazotiert und die Azidogruppe einführt und gegebenenfalls in ihre Salze überführt.

16. Verfahren zur Herstellung von Additionssalzen der Formel I gemäss einem der Ansprüche 12 bis 15, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

17. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder pyrimidinyl-harnstoff der Formel I, Anspruch 1, enthält.

18. Die Verwendung der N-Phenylsulfonyl-N'-triazinyl- oder -pyrimidinyl-harnstoffe der Formel I, gemäss Anspruch 1, oder sie enthaltender Mittel zur Bekämpfung unerwünschten Pflanzenwachstums.

19. Die Verwendung der N-Phenylsulfonyl-N'-triazinyl- oder -pyrimidinyl-harnstoffe der Formel I, gemäss Anspruch 1, oder sie enthaltender Mittel zur Regulierung des Pflanzenwachstums.

20. Die Verwendung der N-Phenylsulfonyl-N'-triazinyl- oder -pyrimidinyl-harnstoffe der Formel I, oder sie

enthaltender Mittel, gemäss Anspruch 18, zur selektiven pre- oder postemergenten Bekämpfung von Unkräutern in Nutzpflanzenkulturen.

21. Die Verwendung gemäss Anspruch 20 in Zuckerrohr-, Getreide-, Mais-, Reis-, Soja- und Baumwollkulturen.

22. Die Verwendung der N-phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

23. Die Verwendung gemäss Anspruch 22 in Sojakulturen.

24. Die Verwendung gemäss Anspruch 19 bei Bodendecker-Leguminosen.

**Patentansprüche** für den Vertragsstaat AT.

1. Ein herbizides und den Pflanzenwuchs regulierendes Mittel, dadurch gekennzeichnet, dass es neben Träger- und/oder anderen Zuschlagstoffen als Wirkstoff mindestens einen N-phenylsulfonyl-N'-triazinyl- oder pyrimidinyl-harnstoff der Formel I

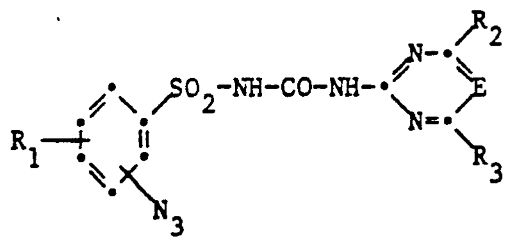

(I),

oder ein Salz dieser Verbindungen enthält, worin

E Stickstoff oder die Methinbrücke,

$R_1$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_1$-$C_4$-Alkylsulfinyl, $C_1$-$C_4$-Alkylsulfonyl oder $C_2$-$C_5$-Alkoxyalkoxy,

$R_2$ $C_1$-$C_3$-Alkyl, $C_1$-$C_3$-Alkoxy oder $C_1$-$C_3$-Halogenalkoxy,

$R_3$ Wasserstoff, Halogen, $C_1$-$C_3$-Alkyl, $C_1$-$C_3$-Halogenalkyl, $C_1$-$C_3$-Alkoxy, $C_1$-$C_3$-Halogenalkoxy, $C_2$-$C_5$-Alkoxyalkoxy oder $-NR_4R_5$ bedeuten, wobei

$R_4$ für Wasserstoff oder Methyl und

$R_5$ für Wasserstoff, Methyl, Äthyl oder Methoxy stehen.

2. Mittel gemäss Anspruch 1, dadurch gekennzeichnet, dass der Azidorest in 2-Stellung zur Sulfonylgruppe steht.

3. Mittel gemäss Anspruch 1, dadurch gekennzeichnet, dass $R_1$ Wasserstoff ist.

4. Mittel gemäss Anspruch 1, dadurch gekennzeichnet, dass $R_2$ und $R_3$ zusammen nicht mehr als 3 Kohlenstoffatome enthalten.

5. Mittel gemäss Anspruch 1, dadurch gekennzeichnet, dass $R_2$ und $R_3$ ausgewählt sind aus der Gruppe Methyl, Methoxy, Dimethylamino, Difluormethoxy und 2,2,2-Trifluoräthoxy.

6. Mittel gemäss Anspruch 1, dadurch gekennzeichnet, dass $R_1$ $C_1$-$C_4$-Alkoxycarbonyl bedeutet.

7. Mittel gemäss Anspruch 1, dadurch gekennzeichnet, dass der Rest $R_1$ in 2-Stellung zur Sulfonylgruppe steht und $C_1$-$C_4$-Alkoxycarbonyl bedeutet und $R_2$ und $R_3$ ausgewählt sind aus der Gruppe Methyl, Methoxy, Difluormethoxy, Dimethylamino und 2,2,2-Trifluoräthoxy und zusammen nicht mehr als 3 Kohlenstoffatome enthalten.

8. Verbindungen gemäss Anspruch 7, dadurch gekennzeichnet, dass $R_2$ und $R_3$ für Methyl oder Methoxy stehen.

9. Mittel gemäss Anspruch 1, dadurch gekennzeichnet, dass es einen Wirkstoff enthält, ausgewählt aus der Gruppe N-(2-Azidophenyl-sulfonyl)-N'-(4-methoxy-6-methyl-1,3,5-triazin-2-yl)-harnstoff,

N-(2-Methoxycarbonyl-5-azidophenylsulfonyl)-N'-(4-difluormethoxy-6-methylpyrimidin-2-yl)-harnstoff und

N-(2-Azidophenylsulfonyl)-N'-(4-methoxy-6-methyl-pyrimidin-2-yl)-harnstoff.

10. Verfahren zur Herstellung der Verbindungen der Formel I, Anspruch 1, dadurch gekennzeichnet, dass man entweder

a) ein Azidophenylsulfonamid der Formel II

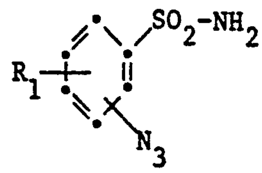

(II),

worin $R_1$ die unter Formel I gegebene Bedeutung hat, in Gegenwart einer Base mit einem N-Pyrimidinyl- oder -triazinylcarbamat der Formel III

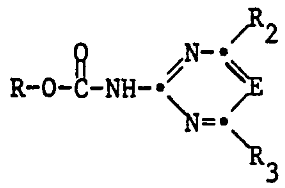

(III)

worin E, $R_2$ und $R_3$ die unter Formel I gegebene Bedeutung haben und R für Phenyl, Alkyl oder substituiertes Phenyl steht, umsetzt oder

b) ein Azidophenylsulfonylisocyanat der Formel IV

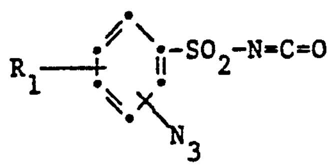

(IV),

worin $R_1$ die unter Formel I gegebene Bedeutung hat, gegebenenfalls in Gegenwart einer Base, mit einem Amin der Formel V

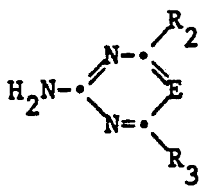

(V),

worin $R_2$ und $R_3$ die unter Formel I gegebene Bedeutung haben, umsetzt oder

c) ein N-Azidophenylsulfonylcarbamat der Formel VI

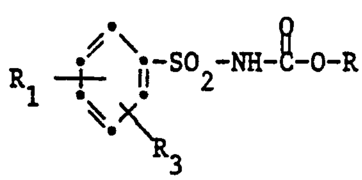

(VI),

worin $R_1$ die unter Formel I gegebene Bedeutung hat und R für Phenyl, Alkyl oder substituiertes Phenyl steht, mit einem Amin der oben angegebenen Formel V umsetzt oder

d) einen Aminosulfonylharnstoff der Formel VII

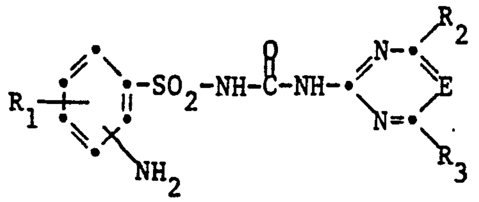

(VII),

worin E, $R_1$, $R_2$ und $R_3$ die unter Formel I gegebene Bedeutungen haben, diazotiert und die Azidogruppe einführt; und gegebenenfalls in ihre Salze überführt indem man einen Sulfonylharnstoff der Formel I mit einem Amin, einem Alkalimetall- oder Erdalkalimetallhydroxid oder einer quaternären Ammoniumbase umsetzt.

11. Die Verwendung der N-Phenylsulfonyl-N'-triazinyl- oder -pyrimidinyl-harnstoffe der Formel I, gemäss Anspruch 1, oder sie enthaltender Mittel zur Bekämpfung unerwünschten Pflanzenwachstums.

12. Die Verwendung der N-Phenylsulfonyl-N'-triazinyl- oder -pyrimidinyl-harnstoffe der Formel I, gemäss Anspruch 1, oder sie enthaltender Mittel zur Regulierung des Pflanzenwachstums.

13. Die Verwendung der N-Phenylsulfonyl-N'-triazinyl- oder -pyrimidinyl-harnstoffe der Formel I, oder sie enthaltender Mittel, gemäss Anspruch 11 zur selektiven pre- oder postemergenten Bekämpfung von Unkräutern in Nutzpflanzenkulturen.

14. Die Verwendung gemäss Anspruch 13 in Zuckerrohr-, Getreide-, Mais-, Reis-, Soja- und Baumwollkulturen.

15. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

16. Die Verwendung gemäss Anspruch 15 in Sojakulturen.

17. Die Verwendung gemäss Anspruch 12 bei Bodendecker-Leguminosen.

**Claims** for the Contracting States: BE, CH, DE, FR, GB, IT, LI, NL, SE.

1. N-azidophenylsulfonyl-N'-pyrimidinyl- and -N'-triazinylureas of the formula I

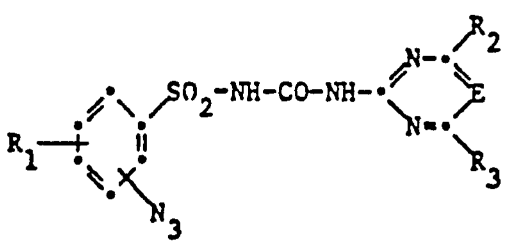

(I),

wherein

E is nitrogen or the methine bridge,

$R_1$ is hydrogen, halogen, nitro, $C_1$-$C_4$alkyl, $C_1$-$C_4$-haloalkyl, $C_1$-$C_4$alkoxy, $C_1$-$C_4$haloalkoxy, $C_1$-$C_4$-alkoxycarbonyl, $C_1$-$C_4$alkylthio, $C_1$-$C_4$alkylsulfinyl, $C_1$-$C_4$alkylsulfonyl or $C_2$-$C_5$alkoxyalkoxy,

$R_2$ is $C_1$-$C_3$alkyl, $C_1$-$C_3$alkoxy or $C_1$-$C_3$haloalkoxy,

$R_3$ is hydrogen, halogen, $C_1$-$C_3$alkyl, $C_1$-$C_3$haloalkyl, $C_1$-$C_3$alkoxy, $C_1$-$C_3$haloalkoxy, $C_2$-$C_5$alkoxyalkoxy or $-NR_4R_5$, wherein

$R_4$ is hydrogen or methyl and

$R_5$ is hydrogen, methyl, ethyl or methoxy, and the salts of these compounds.

2. Compounds according to claim 1, characterised in that the azido radical is in the 2-position to the sulfonyl group.

3. Compounds according to claim 1, characterised in that $R_1$ is hydrogen.

4. Compounds according to claim 1, characterised in that $R_2$ and $R_3$ together contain not more than 3 carbon atoms.

5. Compounds according to claim 1, characterised in that $R_2$ and $R_3$ are selected from the group consisting of methyl, methoxy, dimethylamino, difluoromethoxy and 2,2,2-trifluoroethoxy.

6. Compounds according to claim 1, characterised in that $R_1$ is $C_1$-$C_4$alkoxycarbonyl.

7. Compounds according to claim 1, characterised in that the radical $R_1$ is in the 2-position to the sulfonyl group and is $C_1$-$C_4$alkoxycarbonyl and $R_2$ and $R_3$ are selected from the group consisting of methyl, methoxy, difluoromethoxy, dimethylamino and 2,2,2-trifluoroethoxy and together contain not more than 3 carbon atoms.

8. Compounds according to claim 7, characterised in that $R_2$ and $R_3$ are methyl, methoxy or difluoromethoxy.

9. N-(2-methoxycarbonyl-5-azidophenylsulfonyl)-N'-(4-difluoromethoxy-6-methylpyrimidin-2-yl)urea according to claim 1.

10. N-(2-azidophenylsulfonyl)-N'-(4-methoxy-6-methyl-1,3,5-triazin-2-yl)urea according to claim 1.

11. N-(2-azidophenylsulfonyl)-N'-(4-methoxy-6-methyl-pyrimidin-2-yl)urea according to claim 1.

12. A process for the preparation of the compounds of the formula I, claim 1, characterised in that an azidophenylsulfonamide of the formula II

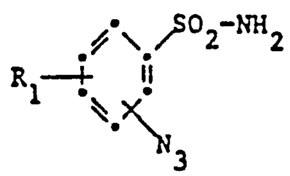

(II),

wherein $R_1$ is as defined for formula I, is reacted with an N-pyrimidinyl- or N-triazinyl-carbamate of the formula III

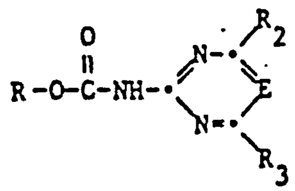

(III),

wherein E, $R_2$ and $R_3$ are as defined for formula I and R is phenyl, alkyl or substituted phenyl, in the presence of a base, and, if desired, the compound so obtained is converted into a salt thereof.

13. A process for the preparation of the compounds of the formula I, claim 1, characterised in that an azidophenylsulfonylisocyanate of the formula IV

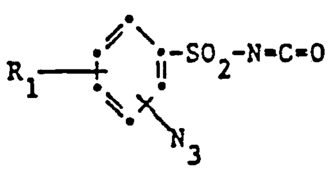

(IV),

wherein $R_1$ is as defined for formula I, is reacted with an amine of the formula V

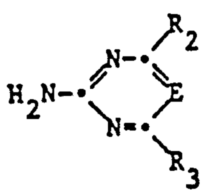

(V),

wherein $R_2$ and $R_3$ are as defined for formula I, optionally in the presence of a base, and, if desired, the compound so obtained is converted into a salt thereof.

14. A process for the preparation of the compounds of the formula I, claim 1, characterised in that an N-azidophenylsulfonylcarbamate of the formula VI

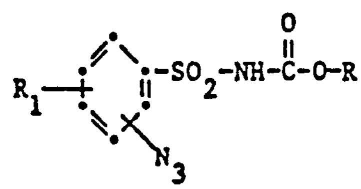

(VI),

wherein $R_1$ is as defined for formula I and R is phenyl, alkyl or substituted phenyl, is reacted with an amine of the formula V above, and, if desired, the compound so obtained is converted into a salt thereof.

15. A process for the preparation of the compounds of the formula I, claim 1, characterised in that an aminosulfonylurea of the formula VII

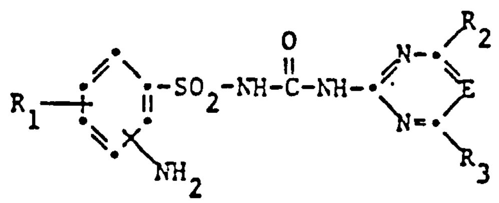

(VII),

wherein E, $R_1$, $R_2$ and $R_3$ are as defined for formula I, is diazotised and the azido group is introduced, and, if desired, the compound so obtained is converted into a salt thereof.

16. A process for the preparation of addition salts of the formula I according to any one of claims 12 to 15, characterised in that a sulfonylurea of the formula I is reacted with an amine, an alkali metal hydroxide or alkaline earth metal hydroxide or with a quaternary ammonium base.

17. A herbicidal and plant growth regulating composition, characterised in that it contains, as active ingredient, at least one N-phenylsulfonyl-N'-triazinyl- or -N'-pyrimidinylurea of the formula I, claim 1, together with carriers and/or other adjuvants.

18. The use of the N-phenylsulfonyl-N'-triazinylureas or N-phenylsulfonyl-N'-pyrimidinylureas of the formula I, according to claim 1, or of compositions containing such compounds, for controlling undesired plant growth.

19. The use of the N-phenylsulfonyl-N'-triazinylureas or N-phenylsulfonyl-N'-pyrimidinylureas of the formula I, according to claim 1, or of compositions containing such compounds, for regulating plant growth.

20. The use of the N-phenylsulfonyl-N'-triazinylureas or N-phenylsulfonyl-N'-pyrimidinylureas of the formula I, or of compositions containing such compounds, according to claim 18, for selectively controlling weeds pre- or postemergence in crops of useful plants.

21. The use according to claim 20 in crops of sugar cane, cereals, maize, rice, soybeans and cotton.

22. The use of the N-phenylsulfonyl-N'-triazinylureas or N-phenylsulfonyl-N'-pyrimidinylureas of the formula I, claim 1, or of compositions containing such compounds, for regulating the growth of cultivated plants to obtain an increase in yield.

23. The use according to claim 22 in crops of soybeans.

24. The use according to claim 19 in cover crop leguminosae.

**Claims** for the Contracting State: AT.

1. A herbicidal and plant growth regulating composition, characterised in that it contains, as active ingredient, together with carriers and/or other adjuvants, at least one N-phenylsulfonyl-N'-triazinyl- or -N'-pyrimidinylurea of the formula I

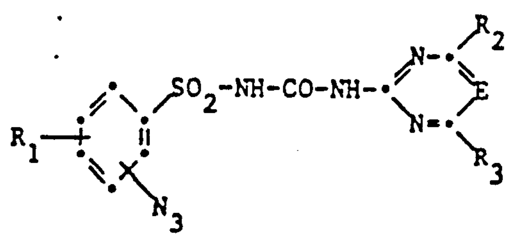

(I),

or a salt of these compounds,
wherein

E is nitrogen or the methine bridge,

$R_1$ is hydrogen, halogen, nitro, $C_1$-$C_4$alkyl, $C_1$-$C_4$-haloalkyl, $C_1$-$C_4$alkoxy, $C_1$-$C_4$haloalkoxy, $C_1$-$C_4$-alkoxycarbonyl, $C_1$-$C_4$alkylthio, $C_1$-$C_4$alkylsulfinyl, $C_1$-$C_4$alkylsulfonyl or $C_2$-$C_5$alkoxyalkoxy,

$R_2$ is $C_1$-$C_3$alkyl, $C_1$-$C_3$alkoxy or $C_1$-$C_3$haloalkoxy,

$R_3$ is hydrogen, halogen, $C_1$-$C_3$alkyl, $C_1$-$C_3$haloalkyl, $C_1$-$C_3$alkoxy, $C_1$-$C_3$haloalkoxy, $C_2$-$C_5$alkoxyalkoxy or $-NR_4R_5$ wherein

$R_4$ is hydrogen or methyl and

$R_5$ is hydrogen, methyl, ethyl or methoxy.

2. A composition according to claim 1, characterised in that the azido radical is in the 2-position to the sulfonyl group.

3. A composition according to claim 1, characterised in that $R_1$ is hydrogen.

4. A composition according to claim 1, characterised in that $R_2$ and $R_3$ together contain not more than 3 carbon atoms.

5. A composition according to claim 1, characterised in that $R_2$ and $R_3$ are selected from the group consisting of methyl, methoxy, dimethylamino, difluoromethoxy and 2,2,2-trifluoroethoxy.

6. A composition according to claim 1, characterised in that $R_1$ is $C_1$-$C_4$alkoxycarbonyl.

7. A composition according to claim 1, characterised in that the radical $R_1$ is in the 2-position to the sulfonyl group and is $C_1$-$C_4$alkoxycarbonyl and $R_2$ and $R_3$ are selected from the group consisting of methyl, methoxy, difluoromethoxy, dimethylamino and 2,2,2-trifluoroethoxy and together contain not more than 3 carbon atoms.

8. Compounds according to claim 7, characterised in that $R_2$ and $R_3$ are methyl or methoxy.

9. A composition according to claim 1, characterised in that it contains an active ingredient selected from the group consisting of N-(2-azidophenylsulfonyl)-N'-(4-methoxy-6-methyl-1,3,5-triazin-2-yl)urea, N-(2-methoxycarbonyl-5-azidophenylsulfonyl)-N'-(4-difluoromethoxy-6-methylpyrimidin-2-yl)urea and N-(2-azidophenylsulfonyl)-N'-(4-methoxy-6-methylpyrimidin-2-yl)urea.

10. A process for the preparation of the compounds of the formula I, claim 1, characterised in that either

a) an azidophenylsulfonamide of the formula II

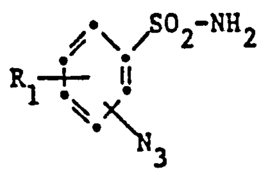

(II),

wherein $R_1$ is as defined for formula I, is reacted with an N-pyrimidinyl- or N-triazinyl-carbamate of the formula III

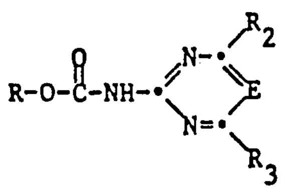

(III),

wherein E, $R_2$ and $R_3$ are as defined for formula I and R is phenyl, alkyl or substituted phenyl, in the presence of a base, or

b) an azidophenylsulfonylisocyanate of the formula IV

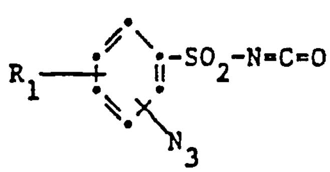

(IV),

wherein $R_1$ is as defined for formula I, is reacted with an amine of the formula V

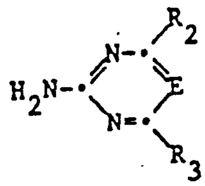 (V),

wherein $R_2$ and $R_3$ are as defined for formula I, optionally in the presence of a base, or

c) an N-azidophenylsulfonylcarbamate of the formula VI

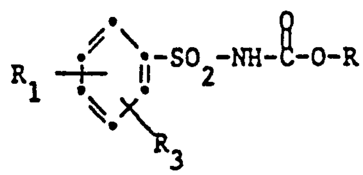 (VI),

wherein $R_1$ is as defined for formula I and R is phenyl, alkyl or substituted phenyl, is reacted with an amine of the formula V above, or

d) an aminosulfonylurea of the formula VII

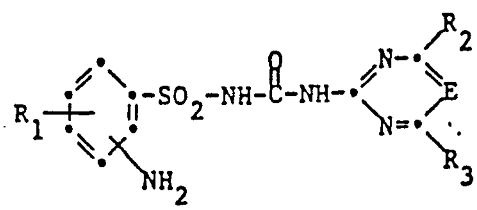 (VII)

wherein E, $R_1$, $R_2$ and $R_3$ are as defined for formula I is diazotised and the azido group is introduced; and, if desired, the compound so obtained is converted into a salt thereof by reacting a sulfonylurea of the formula I with an amine, an alkali metal hydroxide or alkaline earth metal hydroxide or with a quaternary ammonium base.

11. The use of the N-phenylsulfonyl-N'-triazinylureas or N-phenylsulfonyl-N'-pyrimidinylureas of the formula I, according to claim 1, or of compositions containing such compounds, for controlling undesired plant growth.

12. The use of the N-phenylsulfonyl-N'-triazinylureas or N-phenylsulfonyl-N'-pyrimidinylureas of the formula I, according to claim 1, or of compositions containing such compounds, for regulating plant growth.

13. The use of the N-phenylsulfonyl-N'-triazinylureas or N-phenylsulfonyl-N'-pyrimidinylureas of the formula I, or of compositions containing such compounds, according to claim 11, for selectively controlling weeds pre- or postemergence in crops of useful plants.

14. The use according to claim 13 in crops of sugar cane, cereals, maize, rice, soybeans and cotton.

15. The use of the N-phenylsulfonyl-N'-triazinylureas or N-phenylsulfonyl-N'-pyrimidinylureas of the formula I, claim 1, or of compositions containing such compounds, for regulating the growth of cultivated plants to obtain an increase in yield.

16. The use according to claim 15 in crops of soybeans.

17. The use according to claim 12 in cover crop leguminosae.

**Revendications** pour les Etats contractants: BE, CH, DE, FR, GB, IT, LI, NL, SE.

1. N-azidophénylsulfonyl-N'-pyrimidinyl- et -triazinyl-urées de formule I

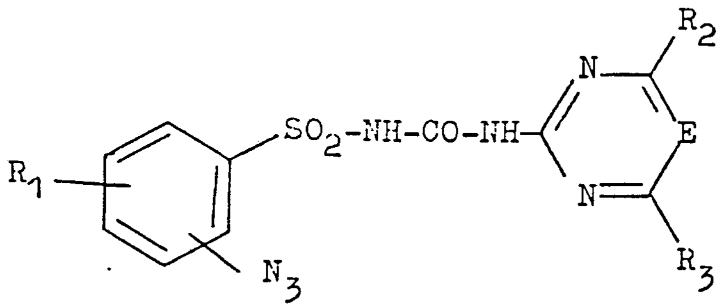

(I),

dans laquelle

E représente l'azote ou le pont méthine,

$R_1$ représente l'hydrogène, un halogène, un groupe nitro, alkyle en $C_1$-$C_4$, halogénoalkyle en $C_1$-$C_4$, alcoxy en $C_1$-$C_4$, halogénoalcoxy en $C_1$-$C_4$, (alcoxy en $C_1$-$C_4$)-carbonyle, alkylthio en $C_1$-$C_4$-alkylsulfinyle en $C_1$-$C_4$, alkylsulfonyle en $C_1$-$C_4$ ou alcoxyalcoxy en $C_2$-$C_5$,

$R_2$ représente un groupe alkyle en $C_1$-$C_3$, alcoxy en $C_1$-$C_3$ ou halogénoalcoxy en $C_1$-$C_3$,

$R_3$ représente l'hydrogène, un halogène, un groupe alkyle en $C_1$-$C_3$, halogénoalkyle en $C_1$-$C_3$, alcoxy en $C_1$-$C_3$, halogénoalcoxy en $C_1$-$C_3$, alcoxyalcoxy en $C_2$-$C_5$ ou $-NR_4R_5$ dans lequel

$R_4$ représente l'hydrogène ou un groupe méthyle, et

$R_5$ représente l'hydrogène, un groupe méthyle, éthyle ou méthoxy,

et les sels de ces composés.

2. Composés selon la revendication 1, caractérisés en ce que le groupe azido est en position 2 par rapport au groupe sulfonyle.

3. Composés selon la revendication 1, caractérisés en ce que $R_1$ représente l'hydrogène.

4. Composés selon la revendication 1, caracterises en ce que $R_2$ et $R_3$ contiennent ensemble au maximum 3 atomes de carbone.

5. Composés selon la revendication 1, caractérisés en ce que $R_2$ et $R_3$ sont choisis parmi les groupes méthyle, méthoxy, diméthylamino, difluorométhoxy et 2,2,2-trifluoréthoxy.

6. Composés selon la revendication 1, caractérisés en ce que $R_1$ représente un groupe (alcoxy en $C_1$-$C_4$)-carbonyle.

7. Composés selon la revendication 1, caractérisés en ce que le groupe $R_1$ est en position 2 par rapport au groupe sulfonyle et est un groupe (alcoxy en $C_1$-$C_4$)-carbonyle, et $R_2$ et $R_3$ sont choisis parmi les groupes méthyle, méthoxy, difluorométhoxy, diméthylamino et 2,2,2-trifluoréthoxy et contiennent ensemble au maximum 3 atomes de carbone.

8. Composés selon la revendication 7, caractérisé en ce que $R_2$ et $R_3$ représentent des groupes méthyle, méthoxy ou difluorométhoxy.

9. La N-(2-méthoxycarbonyl-5-azidophénylsulfonyl)-N'-(4-difluorométhoxy-6-méthylpyrimidine-2-yl)-urée selon la revendication 1.

10. La N-(2-azidophénylsulfonyl)-N'-(4-méthoxy-6-méthyl-1,3,5-triazine-2-yl)-urée selon la revendication 1.

11. La N-(2-azidophénylsulfonyl)-N'-(4-méthoxy-6-méthylpyrimidine-2-yl)-urée selon la revendication 1.

12. Procédé de préparation des composés de formule I, revendication 1, caractérisé en ce que l'on fait réagir un azidophénylsulfonamide de formule II

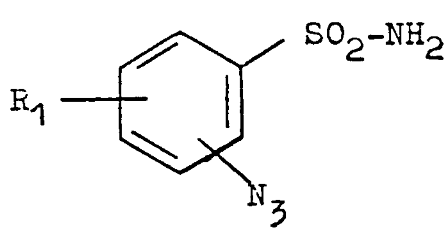

(II)

dans la quelle $R_1$ a les significations indiquées en référence à la formule I, en présence d'une base, avec un N-pyrimidinyl- ou -triazinyl-carbamate de formule III.

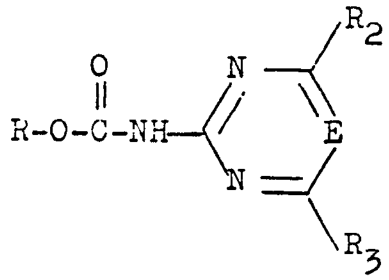

(III)

dans laquelle E, $R_2$ et $R_3$ ont les significations indiquées en référence à la formule I et R représente un groupe phényle, alkyle ou phényle substitué, et le cas échéant on les convertit en leurs sels.

13. Procédé de préparation des composés de formule I, revendication 1, caractérisé, en ce que l'on fait réagir un azidophénylsulfonylisocyanate de formule IV

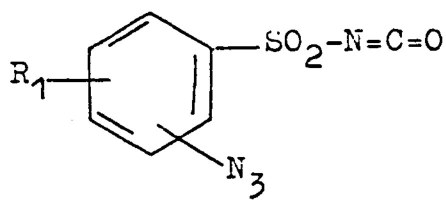

(IV),

dans laquelle $R_1$ a les significations indiquées en référence à la formule I, éventuellement en présence d'une base, avec une amine de formule V

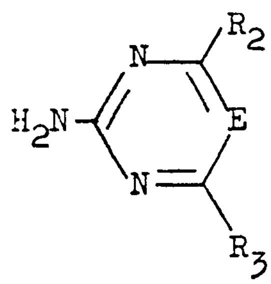

(V),

dans laquelle $R_2$ et $R_3$ ont les significations indiquées en référence à la formule I et le cas échéant, on les convertit en leurs sels.

14. Procédé de préparation des composés de formule I, revendication 1, caractérisé en ce que l'on fait réagir un N-azidophénylsulfonylcarbamate de formule VI

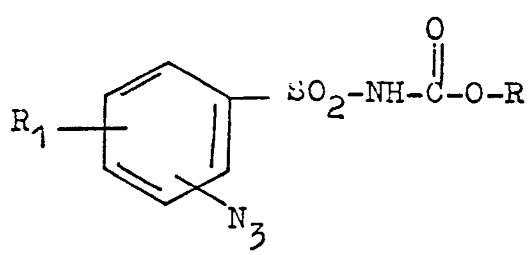

(VI)

dans laquelle $R_1$ a les significations indiquées en référence à la formule I et R représente un groupe phényle, alkyle ou phényle substitué, avec une amine de formule V ci-dessus et le cas échéant on les convertit en leurs sels.

15. Procedé de préparation des composés de formule I, revendication 1, caractérisé en ce que l'on diazote une aminosulfonylurée de formule VII

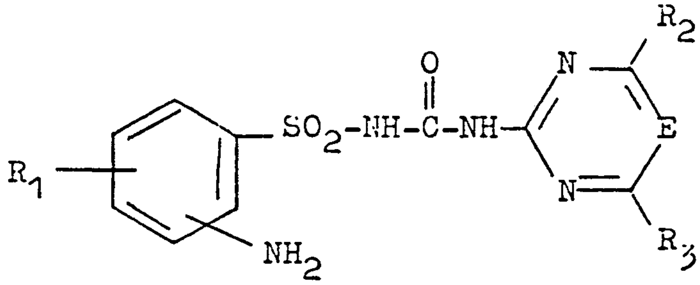

(VII),

dans laquelle E, $R_1$, $R_2$ et $R_3$ ont les significations indiquées en référence à la formule I, on introduit le groupe azido et le cas échéant on les convertit en leurs sels.

16. Procédé de préparation des sels d'addition de formule I selon l'une des revendications 12 à 15, caractérisé en ce que l'on fait réagir une sulfonylurée de formule I avec une amine, un hydroxyde de métal alcalin ou alcalino-terreux ou une base d'ammonium quaternaire.

17. Un produit herbicide et régulateur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, revendication 1.

18. L'utilisation des N-phénylsulfonyl-N'-triazinyl- ou -pyrimidinyl-urées de formule I, selon la revendication 1, ou de produits en contenant, pour la lutte contre la croissance des végétaux indésirables.

19. L'utilisation des N-phénylsulfonyl-N'triazinyl- ou -pyrimidinyl-urées de formule I, selon la revendication 1, ou de produits en contenant, pour la régulation de la croissance des végétaux.

20. L'utilisation des N-phénylsulfonyl-N'triazinyl- ou -pyrimidinyl-urées de formule I, ou de produits en contenant, selon la revendication 18, pour la lutte sélective en pré-levée ou en post-levée contre les végétaux adventices dans les cultures de végétaux utiles.

21. L'utilisation selon la revendication 20, dans les cultures de canne à sucre, de céréales, de maïs, de riz, de soja et de coton.

22. L'utilisation des N-phénylsulfonyl-N'triazinyl- ou -pyrimidinyl-urées de formule I, revendication 1, ou de produits en contenant, pour la régulation de la croissance des végétaux cultivés dans le but d'augmenter la récolte.

23. L'utilisation selon la revendication 22 dans les cultures de soja.

24. L'utilisation selon la revendication 19 sur les légumineuses de couvertures de sol.

**Revendications** pour l'Etat contractant: AT.

1. Un produit herbicide et régulateur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I

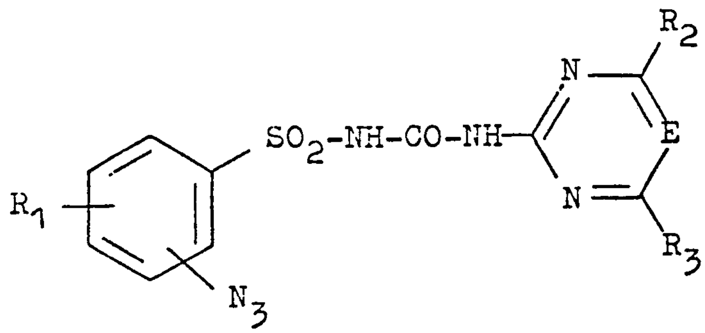

(I),

ou un sel d'un tel composé, dans lesquels

E représente l'azote ou le pont méthine,

$R_1$ représente l'hydrogène, un halogène, un groupe nitro, alkyle en $C_1$-$C_4$, halogénoalkyle en $C_1$-$C_4$, alcoxy en $C_1$-$C_4$, halogénoalcoxy en $C_1$-$C_4$, (alcoxy en $C_1$-$C_4$)-carbonyle, alkylthio en $C_1$-$C_4$, alkylsulfinyle en $C_1$-$C_4$, alkylsulfonyle en $C_1$-$C_4$ ou alcoxyalcoxy en $C_2$-$C_5$,

$R_2$ représente un groupe alkyle en $C_1$-$C_3$, alcoxy en $C_1$-$C_3$ ou halogénoalcoxy en $C_1$-$C_3$,

$R_3$ représente l'hydrogène, un halogène, un groupe alkyle en $C_1$-$C_3$, halogénoalkyle en $C_1$-$C_3$, alcoxy en $C_1$-$C_3$,

halogénoalcoxy en $C_1$-$C_3$, alcoxyalcoxy en $C_2$-$C_5$ ou $-NR_4R_5$ dans lequel
$R_4$ représente l'hydrogène ou un groupe méthyle, et
$R_5$ représente l'hydrogène, un groupe méthyle, éthyle ou méthoxy.

2. Produit selon la revendication 1, caractérisé en ce que le groupe azido est en position 2 par rapport au groupe sulfonyle.

3. Produit selon la revendication 1, caractérisé en ce que $R_1$ représente l'hydrogène.

4. Produit selon la revendication 1, caractérisé en ce que $R_2$ et $R_3$ contiennent ensemble au maximum 3 atomes de carbone.

5. Produit selon la revendication 1, caractérisé en ce que $R_2$ et $R_3$ sont choisis parmi les groupes méthyle, méthoxy, diméthylamino, difluorométhoxy et 2,2,2-trifluoréthoxy.

6. Produit selon la revendication 1, caractérisé en ce que $R_1$ représente un groupe (alcoxy en $C_1$-$C_4$)-carbonyle.

7. Produit selon la revendication 1, caractérisé en ce que $R_1$ est en position 2 par rapport au groupe sulfonyle et représente un groupe (alcoxy en $C_1$-$C_4$)-carbonyle, $R_2$ et $R_3$ sont choisis parmi les groupes méthyle, méthoxy, difluorométhoxy, diméthylamino et 2,2,2-trifluoréthoxy et contiennent ensemble au maximum 3 atomes de carbone.

8. Composés selon la revendication 7, caractérisés en ce que $R_2$ et $R_3$ représentent des groupes méthyle ou méthoxy.

9. Produit selon la revendication 1, caractérisé en ce qu'il contient une substance active choisie dans le groupe formé par la N-(2-azidophényl-sulfonyl)-N'-(4-méthoxy-6-méthyl-1,3,5-triazine-2-yl)-urée, la N-(2-méthoxycarbonyl-5-azidophénylsulfonyl)-N'-(4-difluorométhoxy-6-méthylpyrimidine-2-yl)-urée et la N-(2-azidophénylsulfonyl)-N'-(4-méthoxy-6-méthylpyrimidine-2-yl)-urée.

10. Procédé de préparation des composés de formule I, revendication 1, caractérisé en ce que :

a) ou bien on fait réagir un azidophénylsulfonamide de formule II

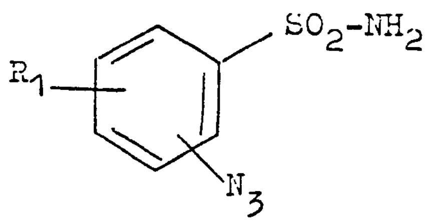

(II),

dans laquelle $R_1$ a les significations indiquées en référence à la formule I, en présence d'une base, avec un N-pyrimidinyl- ou -triazinyl-carbamate de formule III

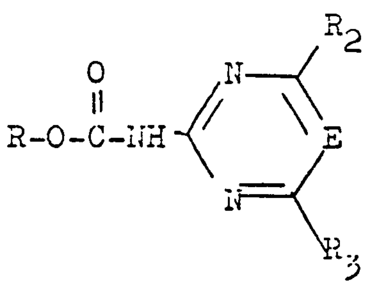

(III)

dans laquelle E, $R_2$ et $R_3$ ont les significations indiquées en référence à la formule I et R représente un groupe phényle, alkyle ou phényle substitué,

b) ou bien on fait réagir un azidophénylsulfonylisocyanate de formule IV

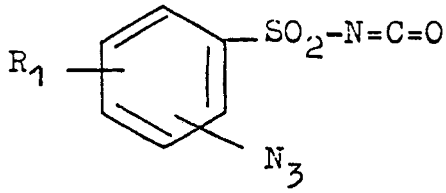

(IV),

dans laquelle $R_1$ a les significations indiquées en référence à la formule I, éventuellement en présence d'une base, avec une amine de formule V

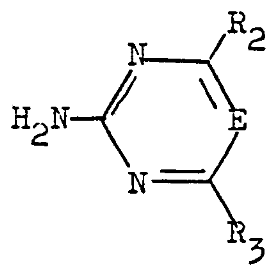

(V),

dans laquelle $R_2$ et $R_3$ ont les significations indiquées en référence à la formule I.
c) ou bien on fait réagir un N-azidophénylsulfonylcarbamate de formule VI

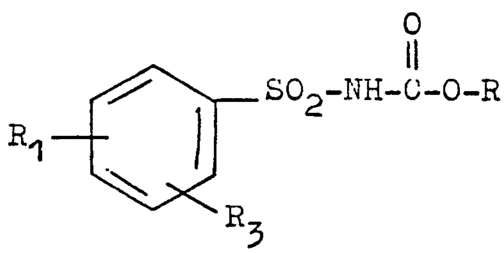

(VI),

dans laquelle $R_1$ a les significations indiquées en référence à la formule I et R représente un groupe phényle, alkyle ou phényle substitué, avec une amine de formule V ci-dessus,
d) ou bien on diazote une aminosulfonylurée de formule VII

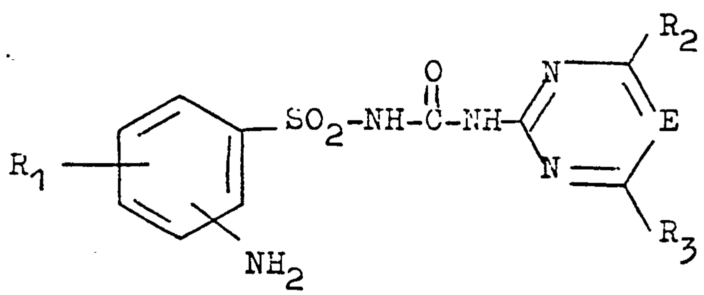

(VII),

dans laquelle E, $R_1$, $R_2$ et $R_3$ ont les significations indiquées en référence à la formule I, et on introduit le groupe azido; et le cas échéant on les convertit en leurs sels, en faisant réagir une sulfonylurée de formule I avec une amine, un hydroxyde de métal alcalin ou alcalino-terreux ou une base d'ammonium quaternaire.

11. L'utilisation des N-phénylsulfonyl-N'triazinyl- ou -pyrimidinyl-urées de formule I selon la revendication 1, ou de produits en contenant dans la lutte contre les croissances de végétaux indésirables.

12. L'utilisation des N-phénylsulfonyl-N'triazinyl- ou pyrimidinyl-urées de formule I selon la revendication 1, ou de produits en contenant, pour la régulation de la croissance des végétaux.

13. L'utilisation des N-phénylsulfonyl-N'triazinyl- ou -pyrimidinyl-urées de formule I ou de produits en contenant selon la revendication 11, pour la lutte sélective en pré-levée ou en post-levée contre les végétaux adventices dans les cultures de végétaux utiles.

14. L'utilisation selon la revendication 13 dans les cultures de canne à sucre, de céréales, de maïs, de riz, de soja et de coton.

15. L'utilisation des N-phénylsulfonyl-N'triazinyl- ou -pyrimidinyl-urées de formule I, revendication 1, ou de produits en contenant pour la régulation de la croissance des végétaux utiles en vue d'une augmentation de récolte.

16. L'utilisation selon la revendication 15 dans les cultures de soja.

17. L'utilisation selon la revendication 12 sur les légumineuses de couvertures de sol.